# EUROPEAN PATENT APPLICATION

(11) **EP 4 616 855 A1**
(43) Date of publication of application: **17.09.2025**
(21) Application number: 24163953.3
(22) Date of filing: 15.03.2024
(51) Int. Cl.: A61K 31/428, A61K 9/00, A61P 25/28

(54) **TALAROZOLE FOR USE IN THE TREATMENT OF A DISEASE CAUSED BY MITOCHONDRIAL COMPLEX IV DEFICIENCY**

(71) Applicant: Heinrich Heine Universität Düsseldorf, 40225 Düsseldorf (DE); Université du Luxembourg, 4365 Esch-sur-Alzette (LU)
(72) Inventor: Prigione, Alessandro, 40225 Düsseldorf (DE); Menacho, Carmen, 40223 Düsseldorf (DE); del Sol Mesa, Antonio, 2410 Strassen (LU); Okawa, Satoshi, Pittsburgh 15213 PA (US)
(74) Representative: Michalski Hüttermann & Partner Patentanwälte mbB

(57) **Abstract**

The present invention relates to talarozole, or a pharmaceutically acceptable salt thereof, for use in the treatment of a disease caused by mitochondrial complex IV deficiency, particularly of SURF 1-dependent Leigh syndrome. The present invention further relates to a pharmaceutical composition comprising as an active ingredient talarozole, or a pharmaceutically acceptable salt thereof, and a pharmaceutically acceptable carrier for use in the treatment of a disease caused by mitochondrial complex IV deficiency.

## Description

The present invention relates to the field of treatment of medical conditions associated with mitochondrial complex IV deficiency.

Mitochondria generate the majority of cellular adenosine triphosphate (ATP) by mitochondrial oxidative phosphorylation (OXPHOS), and genetic alterations affecting mitochondrial proteins can cause severe and often fatal disorders. Mitochondrial diseases represent a large class of inborn errors of metabolism. Leigh syndrome, also denoted Leigh disease, is a severe developmental disorder of the nervous system that can be based on numerous genetic defects, which can be located either on nuclear DNA (inherited according to Mendelian rules) or on mitochondrial DNA (mtDNA, maternal inheritance only). Common to all subtypes is selective damage to dopaminergic neurons in the brainstem and basal ganglia, which may be particularly apparent in the context of metabolic crises. Leigh syndrome causes lactic acidosis and symmetric lesions in the central nervous system (CNS), predominantly of basal ganglia and brainstem, leading to intellectual disability and muscle weakness. The commonly most affected mitochondrial complexes in Leigh syndrome are complex I and complex IV. One of the most frequently mutated nuclear genes in Leigh syndrome is the SURF1 gene (Surfeit locus protein 1, NM_003172.2). SURF1-Leigh syndrome patients typically exhibit increased lactate in the liquor and midbrain neurodegeneration. Leigh syndrome affecting children is currently considered an untreatable neurologic mitochondrial disorder. To date, there are no therapeutic options for Leigh syndrome that influence the pathophysiological process.

Tripathy S, et al. "All-Trans-Retinoic Acid Enhances Mitochondrial Function in Models of Human Liver" Mol Pharmacol. 2016 May;89(5):560-74 (doi: 10.1124/mol.116.103697) disclose that inhibition of all-*trans*-retinoic acid (atRA) metabolism by talarozole, a cytochrome P450 (CYP) 26 specific inhibitor, increased the effects of atRA on mitochondrial biogenesis markers in HepG2 cells and in vivo in mice. It is suggested that atRA regulates mitochondrial function and lipid metabolism and that increasing atRA concentrations in human liver via CYP26 inhibition may increase mitochondrial biogenesis and fatty acid β-oxidation and provide therapeutic benefit in diseases associated with mitochondrial dysfunction.

Jacob Morgan Leatherwood "Investigations on novel CYP26A1/B1 Inhibitor, DX308: Using atRA response as a therapeutic target for traumatic brain injury and Parkinson's disease", University of Montana Graduate Student Theses, 2021, discloses that retinoic acid metabolism blocking agents (RAMBAs) are emerging as new therapeutic interventions with the goal of increasing endogenous atRA brain concentration, for the treatment of traumatic brain injury and Parkinson's disease.

Hummel R, Ulbrich S, Appel D, et al., "Administration of all-trans retinoic acid after experimental traumatic brain injury is brain protective" Br J Pharmacol. 2020; 177:5208-5223 (https://doi.org/10.1111/bph.15259) describe that atRA acts neuroprotective in traumatic brain injury.

Hirano M et al., "Emerging Therapies for Mitochondrial Diseases" Essays Biochem. 2018 July 20; 62(3): 467-481. (doi: 10.1042/EBC20170114) describes that retinoic acid has been used to stimulate the retinoid X receptor-alfa (RXRalfa) in cybrid containing a m.3243A>G mutation, ameliorating the respiratory chain defect.

Inak et al. "Defective metabolic programming impairs early neuronal morphogenesis in neural cultures and an organoid model of Leigh syndrome", Nature Communications (2021) 12:1929 (https://doi.org/10.1038/s41467-021-22117-z) showed that *SURF1* mutations impaired neuronal morphogenesis in induced pluripotent stem cell (iPSC)-derived neurons and cerebral organoids. It was shown that prime defects emerged at the level of neural progenitor cells (NPCs), which were unable to perform the metabolic switch required for neural commitment, and thus showed inefficient neurite outgrowth.

Although some progress in discovering the pathophysiological mechanisms of mitochondrial diseases causing Leigh syndrome was made, major improvements in treatment are still lacking.

Therefore, the object underlying the present invention was to provide a means being usable in the treatment of diseases caused by mitochondrial complex IV deficiency, and particularly in the treatment of Leigh syndrome.

The problem is solved by talarozole, or a pharmaceutically acceptable salt thereof, for use in the treatment of a disease caused by mitochondrial complex IV deficiency.

Surprisingly it was found that talarozole provided a dose-dependent improvement in neurite outgrowth capacity in human neurons having a mutation in the mitochondrial complex IV gene SURF 1, which is the most frequent cause of Leigh syndrome (Leigh disease) in children. These findings indicate that talarozole is usable in the treatment and/or prevention of a disease caused by mitochondrial complex IV deficiency, partucularly in the treatment and/or prevention of of Leigh syndrome.

As used herein, the term "mitochondrial complex IV" refers to a transmembrane protein complex found in bacteria and the mitochondrion of eukaryotes, which also is denoted cytochrome c oxidase and catalyzes the final step in the mitochondrial electron transfer chain. This enzyme is regarded as one of the major regulation sites for oxidative phosphorylation, and its dysfunction is associated with a wide range of human disorders.

As used herein, the term "mitochondrial complex IV deficiency", also referred to as complex IV mitochondrial respiratory chain deficiency, COX deficiency or cytochrome c oxidase deficiency, refers to a defect and/or malfunction in the complex IV enzymatic activity. Such deficiency results in a defect and/or malfunction in the mitochondrial oxidative phosphorylation system causing severe and often fatal disorders. Disorders of the mitochondrial respiratory chain cause heterogeneous clinical manifestations, ranging from isolated myopathy to severe multisystem disease affecting several tissues and organs. Features include hypertrophic cardiomyopathy, hepatomegaly and liver dysfunction, hypotonia, muscle weakness, exercise intolerance, developmental delay, delayed motor development and intellectual disability. A subset of affected individuals manifest Leigh syndrome.

In embodiments, the mitochondrial complex IV deficiency is Leigh syndrome (Leigh disease). In embodiments, talarozole, or the pharmaceutically acceptable salt thereof, is for use in the treatment of Leigh syndrome. Advantageously, talarozole has been shown to be able to reduce key disease-specific features of Leigh syndrome, such as increased lactate released in the media, decreased number of TH neurons and aberrant neuronal branching. It is thus assumed that talarozole is usable in the treatment of Leigh syndrome.

As used herein, the term "Leigh syndrome" (LS), also frequently denoted Leigh disease, refers to a severe developmental disorder of the nervous system that can be based on numerous genetic defects, which can be located either on nuclear DNA (inherited according to Mendelian rules) or on mitochondrial DNA (mtDNA, maternal inheritance only). Common to all subtypes is selective damage to dopaminergic neurons in the brainstem and basal ganglia, which may be particularly apparent in the context of metabolic crises. Leigh syndrome causes lactic acidosis and symmetric lesions in the central nervous system (CNS), predominantly of basal ganglia and brainstem, leading to intellectual disability and muscle weakness.

The commonly most affected mitochondrial complexes in Leigh syndrome are complex I and complex IV. One of the most frequently mutated nuclear genes in Leigh syndrome is the SURF1 gene (Surfeit locus protein 1, NM_003172.2). In embodiments, the mitochondrial complex IV deficiency is based in whole or in part on the presence of at least one mutation or variant in the surfeit locus protein 1 (SURF1) gene. In embodiments, talarozole, or the pharmaceutically acceptable salt thereof, is for use in the treatment of a mitochondrial complex IV deficiency that is based in whole or in part on the presence of at least one mutation or variant in the surfeit locus protein 1 (SURF 1) gene.

In embodiments, the disease caused by mitochondrial complex IV deficiency is SURF1-dependent Leigh syndrome. SURF1 mutations are the most frequent cause of Leigh syndrome. In embodiments, talarozole, or the pharmaceutically acceptable salt thereof, is for use in the treatment of SURF1-dependent Leigh syndrome. It has been shown in SURF1-mutant midbrain organoids, which showed key disease-specific features, such as increased lactate released in the media, decreased number of TH neurons and aberrant neuronal branching, that talarozole reduced the aberrant release of lactate in the media, increased the amount of TH positive dopaminergic neurons, and rescued the overall neuronal branching organization.

As used herein, the term "SURF 1-dependent Leigh syndrome" refers to Leigh syndrome, where the disease is caused by one or more mutation or variant in the surfeit locus protein 1 (SURF1) gene. SURF1-dependent Leigh syndrome patients typically exhibit increased lactate in the liquor and midbrain neurodegeneration.

The term "treatment" as used herein refers to alleviating at least one of the symptoms associated with the disease, decreasing disease-caused mortality, or decelerating, impeding or suspending the progress of the disease. The term "treatment" as used herein does not refer to complete curing of the disease, as it does not change the mutated genetics causing the disease.

As used herein, the term "talarozole" refers to the International Nonproprietary Name (INN) of a compound, CAS number 201410-53-9, formerly R115866, of the following formula (1) or to a pharmaceutically acceptable salt thereof:

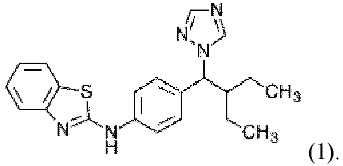

The IUPAC name oftalarozole is N-{4-[2-ethyl-1-(1,2,4-triazol-1-yl)butyl]phenyl}-1,3-benzothiazol-2-amine. The term "talarozole" as used herein encompasses any enantiomers, tautomers, pharmaceutically acceptable salts, as well as solvates, hydrates and solvates of pharmaceutically acceptable salts thereof. In embodiments, the racemate of talarozole may be used or a specific enantiomer of talarozole, such as the (R)- enantiomer.

As used herein, "pharmaceutically acceptable salts" refers to salts prepared from pharmaceutically acceptable non-toxic bases or acids. The term pharmaceutically acceptable salts as used herein includes both acid and base addition salts. Pharmaceutically acceptable salts refer to salts which retain the biological effectiveness and properties of the compound, and which are not biologically or otherwise undesirable. A pharmaceutically acceptable salt can be conveniently prepared from pharmaceutically acceptable non-toxic bases, including inorganic bases and organic bases. The term pharmaceutically acceptable salt includes mineral or organic acid salts of basic residues such as amines, alkali metal salts, organic salts of acidic residues such as carboxylic acids, and addition salts of free acids or free bases. Suitable pharmaceutically acceptable salts include metallic salts and organic salts, such as salts from benzoic acid, citric acid, fumaric acid, maleic acid, and tartaric acid.

In embodiments, the effect of talarozole is an improvement of neuronal growth and connectivity. It has been shown in SURF 1-mutant neural progenitor cells (NPCs) that talarozole provided a dose-dependent rescue of neurite outgrowth capacity. It is assumed that talarozole may improve post-natal brain wiring, which is the process that regulates the establishment of connections between developing neurons in various part of the brain. When this process does not occur properly then a child may experience mental retardation of developmental delay.

Talarozole can be used or included in a composition. Particularly for use as a medicament talarozole can be formulated as a pharmaceutical composition. A further aspect of the present invention relates to a pharmaceutical composition comprising as an active ingredient talarozole, or a pharmaceutically acceptable salt thereof, for use in the treatment of a disease caused by mitochondrial complex IV deficiency. In an embodiment, the pharmaceutical composition comprises talarozole, or a pharmaceutically acceptable salt thereof, and a pharmaceutically acceptable carrier.

The pharmaceutical carrier can be, for example, a solid, liquid, or gas. Suitable carriers and adjuvants can be solid or liquid and correspond to the substances ordinarily employed in formulation technology for pharmaceutical formulations. For example, water, salt solutions such as NaCl, saline, buffered saline, ethanol, alcohols, glycerol, glycols, oils, polyethylene glycols, hydroxymethylcellulose, or combinations thereof and the like may be used to form liquid preparations such as solutions. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen. The pharmaceutical composition can optionally comprise other therapeutic ingredients or adjuvants if desired, such as preservatives, stabilizers, wetting agents, emulsifiers.

The composition can be suitable for oral, dermal, rectal, topical, and parenteral administration. In embodiments, the pharmaceutical composition for use is formulated for topical or local application such as intraneural or periradicular application, or systemic application such as intramuscular, intraperitoneal, intravenous, subcutaneous, intrathecal or oral application.

The pharmaceutical composition can be suitable for oral or parenteral administration. Parenteral administration includes subcutaneous, intramuscular, intravenous, intraneural, periradicular, intraperitoneal, and local administration. Specifically, the pharmaceutical composition may be administered systemically, for example, orally.

The composition can be formulated in accordance with the routine procedures as a pharmaceutical composition adapted for administration to human beings. The pharmaceutical compositions may be conveniently presented in unit dosage form. The pharmaceutical composition may be produced under sterile conditions using standard pharmaceutical techniques well known to those skilled in the art.

In embodiments of the pharmaceutical composition for use, the mitochondrial complex IV deficiency is Leigh syndrome, preferably SURF 1-dependent Leigh syndrome. For the description of mitochondrial complex IV deficiency, Leigh syndrome, and SURF1-dependent Leigh syndrome, reference is made to the description above.

A further aspect relates to a use of talarozole, or a pharmaceutically acceptable salt thereof, for the manufacture of a medicament for the treatment of a disease caused by mitochondrial complex IV deficiency. In embodiments, the mitochondrial complex IV deficiency is Leigh syndrome, preferably SURF 1-dependent Leigh syndrome. For the description of mitochondrial complex IV deficiency, Leigh syndrome, and SURF 1-dependent Leigh syndrome, reference is made to the description above.

A further aspect relates to a method of treating a disease caused by mitochondrial complex IV deficiency, the method comprising the step of administering to a subject a therapeutically effective amount of talarozole, or a pharmaceutically acceptable salt thereof.

The term "therapeutically effective amount" is used herein to mean an amount or dose sufficient to cause an improvement in a clinically significant condition in the subject. For medical purposes, subjects include human subjects and animal subjects, particularly mammalian subjects such as human subjects. The treatment may be a continuous prolonged treatment.

In embodiments, the mitochondrial complex IV deficiency is Leigh syndrome, optionally SURF1-dependent Leigh syndrome. For the description of mitochondrial complex IV deficiency, Leigh syndrome, and SURF1-dependent Leigh syndrome, reference is made to the description above.

Talarozole, or the pharmaceutically acceptable salt thereof, may be administered as a liquid formulation, optionally by oral application, such as in the form of a tablet.

Unless otherwise defined, the technical and scientific terms used herein have the same meaning as commonly understood by one of ordinary skill in the art to which this invention belongs.

The Examples which follow serve to illustrate the invention in more detail but do not constitute a limitation thereof.

The figures show:

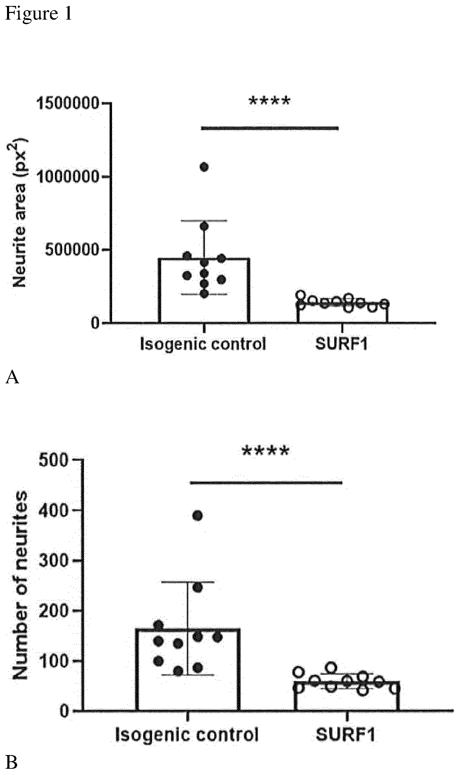

- Figure 1:: the quantification of neurite area in Fig. 1A and number of neurites in Fig. 1B from control and SURF1-mutant neurons. Each value in Figure 1A represents the total neurite area and each value in Figure 1B represents the total number of neurites in each well of a 96-well plate. Significant values were calculated using the non-parametric Mann-Whitney test. *p<0.000 1.

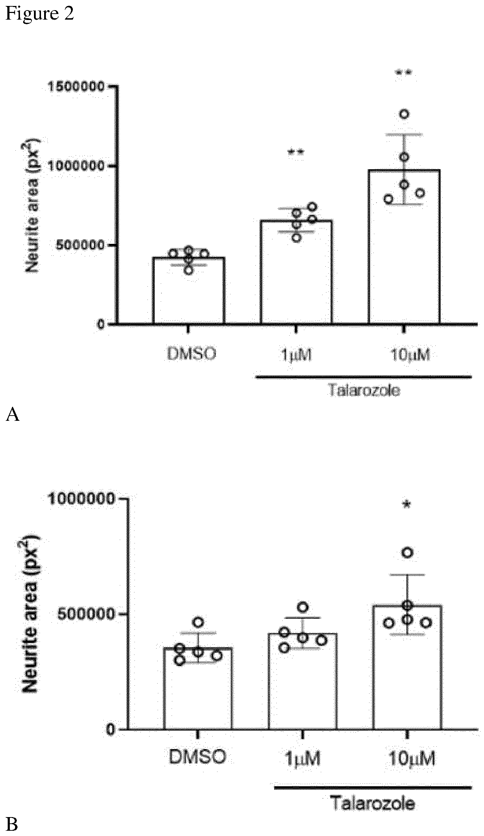

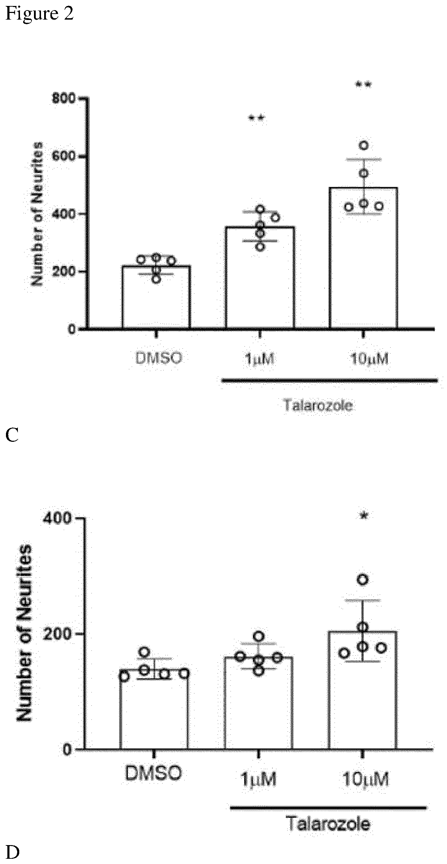

- Figure 2:: the effect of talarozole at concentrations of 1µM and 10µM in 0.1% DMSO for 48h on neurite area (Fig. 2A and B) and number of neurites (Fig. 2C and D) in two independent experiments. Shown are the total values in each well of a 96-well plate, having a total of 5 technical replicates per compounds per experiment. Significant values were calculated using the non-parametric Mann-Whitney test.

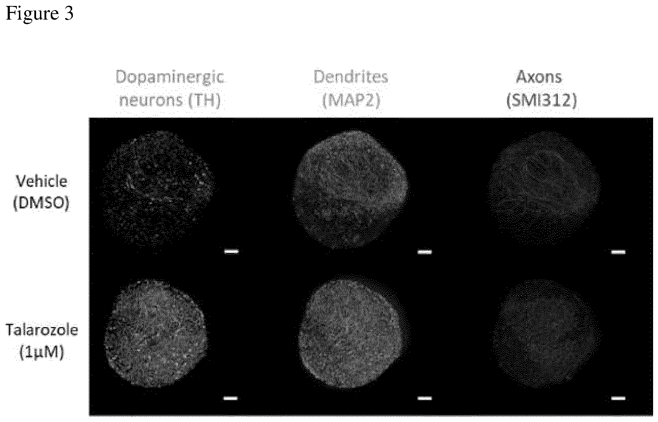

- Figure 3:: the effect of 1 µM talarozole on the amount of dopaminergic neurons (TH staining) and neuronal processes (MAP2 and SMI312 staining, respectively) in midbrain organoids generated from SURF1-mutant NPCs carrying a mutation in the gene SURF1 causative of Leigh syndrome

### Example 1: confirmation of neuromorphogenesis defect in SURF 1-mutant induced neurons

Neuromorphogenesis defect in SURF1-mutant induced neurons were determined in induced neurons (iN) derived from neuronal progenitor cells of control cells and SURF1-mutant cells. CRISPR/Cas9 engineered isogenic SURF1 mutant NPCs were generated as described in Inak et al., Nature Communications (2021) 12:1929. On these cells neural commitment was forced via overexpression of neurogenin 2 (NGN2).

Cell nuclei were stained with Hoechst33142 dye and neurites were stained against microtubule-associated protein 2 (MAP2). Neurite area and number of neurites from control and SURF1-mutant neurons were qunatified. Neurite area in induced neurons (iN) was measured after five days after induction of NGN2 overexpression, using high-content image analysis (HCA) of MAP2-positive stained cells.

Figure 1 shows the results. Each value in Figure 1A represents the total neurite area and each value in Figure 1B represents the total number of neurites in each well of a 96-well plate. Significant values were calculated using the non-parametric Mann-Whitney test. ****p<0.0001. as can be taken from Figure 1, neurite area and number of neurites was significantly lower in SURF 1-mutant induced neurons.

The results confirm that *SURF1* mutations impaired neuronal morphogenesis in induced pluripotent stem cell (iPSC)-derived neurons and cerebral organoids. The prime defects emerged at the level of neural progenitor cells (NPCs), which were unable to perform the metabolic switch required for neural commitment, and thus showed inefficient neurite outgrowth.

### Example 2: Determination of effects of talarozole in SURF 1-mutant induced neurons

CRISPR/Cas9 engineered isogenic SURF1 mutant NPCs, on which neural commitment via overexpression of NGN2 was forced as described above, were used to measure the impact of talarozole on neurite outgrowth capacity. SURF 1-mutant NGN2-derived induced neurons (SURF1_iN) were treated with 0.1% DMSO (vehicle) or 10µM Talarozole. It was seen that talarozole had a positive effect in stimulating neurite development (MAP2+) in SURF1-mutant cells.

The effect oftalarozole at increasing concentrations of 1µM and 10µM in 0.1% DMSO for 48h on neurite area and number of neurites was determined in two independent experiments. Figure 2A and 2B illustrates the effect of talarozole at concentrations of 1µM and 10µM in 0.1% DMSO for 48h on neurite area determined in two independent experiments. Figure 2C and 2D illustrates the effect of talarozole at concentrations of 1µM and 10µM in 0.1% DMSO for 48h on the number of neurites determined in two independent experiments. Shown are the total values in each well of a 96-well plate, having a total of 5 technical replicates per compounds per experiment. Significant values were calculated using the non-parametric Mann-Whitney test.

As can be seen in Figure 2, talarozole exerted a dose-dependent response in increasing both neurite area and number of neurites in SURF1-mutant NGN2-derived induced neurons.

### Example 3: Determination of effects of talarozole in midbrain organoids generated from SURF1-mutant NPCs

The effects of talarozole were further determined in *SURF1*-mutant midbrain organoids (SURF 1-MO), which were generated from SURF1-mutant NPCs as described in Inak et al., Nature Communications (2021) 12: 1929. These oranoids showed key disease-specific features, such as increased lactate released in the media, decreased number of TH neurons and aberrant neuronal branching.

Organoids were treated every 2 days with either vehicle DMSO or 1µM Talarozole for 30 days. The organoids were then fixed and stained with different antibodies against Tyrosine Hydroxylase (TH) which is a marker of dopaminergic neurons, MAP2 which is a marker for neuronal dendrites, and SMI312 which is a marker for neuronal axons.

Figure 3 shows the stained organoids. As can be taken from Figure 3, talarozole treatment increased the amount of dopaminergic neurons (TH) and neuronal processes (MAP2 and SMI312, respectively) in human midbrain organoids carrying a mutation in the gene SURF1 causative of Leigh syndrome.

These results show that talarozole reduced the aberrant release of lactate in the media, increased the amount of TH positive dopaminergic neurons, and rescued the overall neuronal branching organization.

In summary, these findings indicate that talarozole is usable in the treatment of a disease caused by mitochondrial complex IV deficiency, such as Leigh syndrome, and particularly SURF1-dependent Leigh syndrome.

## Claims

1. Talarozole, or a pharmaceutically acceptable salt thereof, for use in the treatment of a disease caused by mitochondrial complex IV deficiency.

2. Talarozole, or the pharmaceutically acceptable salt thereof, for use according to claim 1,
wherein the mitochondrial complex IV deficiency is Leigh syndrome.

3. Talarozole, or the pharmaceutically acceptable salt thereof, for use according to any one of the preceeding claims, wherein the disease caused by mitochondrial complex IV deficiency is SURF1-dependent Leigh syndrome.

4. Talarozole, or the pharmaceutically acceptable salt thereof, for use according to any one of the preceeding claims, wherein the effect of talarozole is an improvement of neuronal growth and connectivity.

5. A pharmaceutical composition comprising as an active ingredient talarozole, or a pharmaceutically acceptable salt thereof, for use in the treatment of a disease caused by mitochondrial complex IV deficiency.

6. The pharmaceutical composition for use according to claim 6, wherein the pharmaceutical composition is formulated for topical or local application such as intraneural or periradicular application, or systemic application such as intramuscular, intraperitoneal, intravenous, subcutaneous, intrathecal or oral application.

7. The pharmaceutical composition for use according to claim 5 or 6, wherein the mitochondrial complex IV deficiency is Leigh syndrome, optionally SURF1-dependent Leigh syndrome.

8. Use of talarozole, or a pharmaceutically acceptable salt thereof, for the manufacture of a medicament for the treatment of a disease caused by mitochondrial complex IV deficiency.

9. The use according to claim 8, wherein the mitochondrial complex IV deficiency is Leigh syndrome, optionally SURF1-dependent Leigh syndrome.

10. A method of treating a disease caused by mitochondrial complex IV deficiency, the method comprising the step of administering to a subject a therapeutically effective amount of talarozole, or a pharmaceutically acceptable salt thereof.

11. The method according to claim 10, wherein the mitochondrial complex IV deficiency is Leigh syndrome, optionally SURF1-dependent Leigh syndrome.
